# EUROPEAN PATENT APPLICATION

(11) **EP 1 610 254 A1**
(43) Date of publication of application: **28.12.2005**
(21) Application number: 04724862.0
(22) Date of filing: 31.03.2004
(51) Int. Cl.: G06F 19/00

(54) **MOLECULAR FUNCTION NETWORK DISPLAY METHOD**

(30) Priority: 31.03.2003 US 458366 P; 11.04.2003 JP 2003107401
(71) Applicant: Institute of Medicinal Molecular Design, Inc., Tokyo 113-0033 (JP)
(72) Inventor: ITAI, Akiko, INST. OF MED. MOLECULAR DESIGN INC., Bunkyo- ku, Tokyo 113-0033 (JP); TOMIOKA, Nobuo, INST. OF MED. MOLECULAR DESIGN INC, Bunkyo-ku, Tokyo 113-0033 (JP); SHIGETAKA, Makoto, INST. OF MED. MOLEC. DESIGN INC, Bunkyo-ku, Tokyo 113-0033 (JP); FUKUDA, Miki, INST. OF MED. MOLECULARDESIGN INC., Bunkyo-ku, Tokyo 113-0033 (JP)
(74) Representative: Sternagel, Fleischer, Godemeyer & Partner
(86) International application number: PCT/JP2004/004704
(87) International publication number: WO 2004/088564

(57) **Abstract**

A graphical user interface that generates a molecule function network with an arbitrary range according to the designation by a user and displays said molecule function network graphically; a graphical user interface with a molecule network window which displays a molecule network included in the molecule function network and an information window which displays one or more information items selected form a group comprising molecules, molecule pairs and bio-events included in said molecule function network, which is **characterized by** that items in the molecule network window and in the information window related with each other are operated interlinked with each other. A molecule function network with information on bio-events including pathological events can be processed and displayed flexibly using a computer.

## Description

### Technical Field

The present invention belongs to a field of biological information processing, and particularly relates to a graphical interface, a program, a device, and a media for displaying and operating information related to a biomolecule and its functions, and information related to a phenomenon in an organism and a disease.

### Background Art

In an organism, various molecules such as amino acids, peptides, lipids, carbohydrates, and small molecules exist along with biomolecules such as nucleic acids and proteins. These biomolecules function by making specific relations such as binding, competition, interaction, and enzyme reaction with other molecules in an organism. Molecules taken from the outside of an organism such as drug molecules or nutrients also participate in or influence functions of the organism by making specific relations with the biomolecules. Such intermolecular relations in an organism can be represented as a graph wherein molecules are shown as apexes and related molecules are linked with edges. Such representation of molecular relations as a graph is hereinafter referred to as a "molecule network."

Mechanisms in an organism at a molecular level can be represented by adding information on the functions that molecules bear in the organism, and information on the phenomenon in the organism (hereinafter referred to as a "bio-event") induced when molecules have a certain relation. A disease can be regarded as a state wherein a certain bio-event is extremely accentuated or suppressed, and a mechanism of a disease at a molecular level can be represented by adding information on the bio-event which changes in a disease (hereinafter referred to as "pathological event") to the molecule network. A molecule network to which information on the functions of the molecules and information on the bio-events (including pathological events) are added is hereinafter referred to as a "molecule-function network." Methods for generating and processing a molecule function network by computer are described in the specifications of PCT/JP01/07830 and PCT/JP03/02847.

A method of displaying a molecule network has been carried out by drawing a diagram of a certain range of the molecule network wherein molecules in the network are arranged appropriately to avoid overlaps. A molecule network can be drawn manually, or it can be drawn and displayed by a computer. Examples of drawing a molecule network by a computer include KEGG, EcoCyc, and GeNet. However, these methods cannot display a molecule function network containing information on the molecule functions and information on the bio-events flexibly. Although the specifications of PCT/JP01/07830 and PCT/JP03/02847 indicate the methods for generating and processing a molecule function network as a data structure, they do not disclose a method of displaying a molecule function network flexibly as a graphic.

### Disclosure of the Invention

An object of the present invention is to provide a method and a system for processing and displaying a molecule function network including the information on bio-events flexibly by using a computer. To be more specific, the object of the present invention is to provide a means that enables a user to examine easily a relation between information on bio-events and a molecule network with a graphic display by generating a molecule function network of an arbitrary range according to a direction by the user.

There are cases where bio-events have relations with each other. Providing a means that enables a user to examine such relations easily is also an object of the present invention. Furthermore, providing a means that enables a user to examine easily not only a molecule function network consisting of biomolecules, but also a molecule function network containing drug molecules and extrinsic molecules, and biomolecules of other species is also an object of the present invention.

After a zealous endeavor to solve the aforementioned objects, the inventors found out that the aforementioned objects can be accomplished by combining a means to generate a molecule function network with an arbitrary range according to the direction of a user, a means to display the molecule function network graphically, and a means to further generate and graphically display a molecule function network according to the user's selection of an arbitrary element in the displayed molecule function network.

According to the present invention, there is provided a graphical user interface wherein a molecule function network with an arbitrary range is generated according to the designation by a user, and the molecule function network is displayed graphically. There is also provided by the present invention, a graphical user interface comprising a molecule network window that displays a molecule network included in the molecule function network and an information window that displays one or more information items selected from a group comprising molecules, molecule pairs, and bio-events included in the molecule function network, and characterized by that items related with each other in the molecule-network window and in the information window are operated interlinked with each other.

According to preferred modes of the present invention, the following graphical user interfaces and methods are provided.
A graphical user interface characterized by that a molecule pair in the molecule-network window and information on the bio-event which occurs due to a quantitative and/or a qualitative fluctuation of the molecule pair or which causes a quantitative and/or a qualitative fluctuation of the molecule pair are displayed in relation to each other, and that the displayed items are operated interlinked with each other;
A graphical user interface characterized by that a molecule in the molecule-network window and information on the bio-event which occurs due to a quantitative and/or a qualitative fluctuation of the molecule or which causes a quantitative and/or a qualitative fluctuation of the molecule are displayed interlinked with each other, and that the displayed items are operated interlinked with each other;
A graphical user interface characterized by that a molecule in the molecule-network window and information on the drug and/or physiologically active molecule which acts on the molecule are displayed in connection with each other, and the displayed items are operated interlinked with each other;
A graphical user interface with a window to display a list of molecules in the molecule-network window, which is characterized by that the molecules in the molecule-network window and items in the list window are operated interlinked with each other;

A graphical user interface with a window to display a list of information items related to molecules and/or molecule pairs in the molecule-network window, which is characterized by that the molecules and/or molecule pairs in the molecule-network and the items in the list window are operated interlinked with each other;
The aforementioned graphical user interface characterized by that the
information on molecules and/or molecule pairs is displayed with categorization or hierarchization;
A graphical user interface with a window to display a list of biological pathways to which molecules and/or molecule pairs in the molecule-network window belong, which is characterized by that the molecules and/or molecule pairs in the molecule-network window and the items in the list window are operated interlinked with each other;
The aforementioned graphical user interface characterized by that the biological pathways are displayed with categorization or hierarchization;
A graphical user interface with a window to display a list of information on bio-events related to molecules and/or molecule pairs in the molecule-network window, which is characterized by that the molecules and/or molecule pairs in the molecule-network window and the items in the list window are operated interlinked with each other;
The aforementioned graphical user interface characterized by that the bio-events are displayed with hierarchization;
The aforementioned graphical user interface which uses Gene Ontology as hierarchized bio-events;
A graphical user interface with a window to display a list of information on pathological events, which is characterized by that items related with each other in the list window are operated interlinked with each other;
The aforementioned graphical user interface characterized by displaying pathological events by category;
The aforementioned graphical user interface characterized by displaying pathological events with hierarchization;
The aforementioned graphical user interface comprising a list of information on quantitative and/or qualitative fluctuations of biomolecules related to the pathological events;
A graphical user interface characterized by that information on the molecule function network is searched by a keyword, and the item hit by the search is highlighted in the molecule network window and/or in the related list window;
A graphical user interface characterized by that one or more molecules and/or molecule pairs are selected in the molecule network window, and a molecule function network is generated and displayed by a connect search by designating the selected molecules and/or molecule pairs as search points;
A method of displaying a molecule function network, which is characterized by displaying molecules and/or molecule pairs differently with symbols and/or colors based on the information on the modification state and/or the activation state;
A method of displaying a molecule function network, which is characterized by displaying molecules and/or molecule pairs differently with symbols and/or colors based on the information on the bio-events;
A method of displaying a molecule function network, which is characterized by displaying molecules and/or molecule pairs differently with symbols and/or colors based on the information on the biomolecules that are the target of actions of physiologically active molecules;
A method of displaying a molecule function network, which is characterized by displaying molecules and/or molecule pairs differently with symbols and/or colors based on the numeric information representing quantitative and/or qualitative states of the molecules and/or the molecule pairs;
The aforementioned method of displaying a molecule function network, which is characterized by using numeric information obtained by any one of the experimental methods comprising DNA microarray, proteomics, and metabolomics;
The aforementioned method of displaying a molecule function network, which is characterized by assigning the numeric information on mRNAs or proteins of a different species based on the ortholog relation;
A method of displaying a molecule function network, which is characterized by displaying edges connecting molecule pairs differently by different drawing methods based on the relation information of the molecule pairs;
A method displaying a molecule function network, which is characterized by that representation of a complex with two or more biomolecules can be switched between a single symbol for the complex and multiple symbols for respective molecules constituting the complex;
A method of displaying a molecule function network, which is characterized by that a bio-event related to a molecule and/or a molecule pair in the molecule network is displayed as an apex, and the molecule and/or molecule pair and the apex are connected with an edge;
A method of displaying a molecule function network, which is characterized by that a biologically active molecule which targets a molecule and/or a molecule pair in the molecule network is displayed as an apex, and the molecule and/or molecule pair and the apex are connected with an edge;
A method of displaying a molecule function network, which is characterized by that a biological pathway and/or another molecule network related to a molecule and/or a molecule pair in the molecule network is displayed as an apex, and the molecule and/or molecule pair and the apex are connected with an edge;
A method of displaying a molecule function network, which is characterized by that a molecule network from two or more search points to a common upstream molecule or to a common downstream molecule is generated by a connect search, and the common upstream molecule or the common downstream molecule is displayed as marked;
A method of displaying a molecule function network wherein a connect search is carried out again within the molecule network generated before by designating one or more molecules as search points, and a partial molecule network generated therein is marked in the original network;
A display grogram of the molecule function network that executes the graphical user interface or the display method described in any one of the above;
A program for displaying a molecule function network which executes graphical user interfaces or methods described above;
A computer-readable media recording the above-mentioned program; and
A device for displaying a molecule function network whereupon the above-mentioned program can be executed.

### Brief Explanation of Drawings

Fig.1 shows an example of the graphical user interface of the present invention.
Fig.2 shows an example of displaying a complex with expansion.
Fig.3 shows an example of displaying a molecule differently by a modification state and/or an activation state.
Fig.4 shows an example of displaying edges connecting molecules differently depending the relation between the molecules.
Fig.5 shows an example of adding bio-events and pathways to a molecule network. "Mucus secretion", "bronchoconstriction" and others connected with broad arrows are the bio-events, and "arachidonic acid cascade" and others connected with broad arrows are the pathways.
Fig.6 shows an example of displaying items of molecule pairs interlinked with each other in the molecule network window and in the information window.
Fig.7 shows an example of displaying items of pathological events interlinked with each other in the molecule network window and in the information window.
Fig.8 shows an example of displaying items of drug molecule information interlinked with each other in the molecule network window and in the information window.
Fig.9 shows an example of displaying a partitioned graph of a molecule network for the expression control of proteins by transcription factors.
Fig. 10 shows an example wherein a connect search is carried out by designating one molecule in the molecule network as the starting point, and the molecule network is expanded.
Fig.11 shows an example wherein common upstream molecules are searched from two molecules and are marked in the molecule network. The molecules of the starting points are marked with the * marks, and the common upstream molecules are marked with the # marks, respectively.
Fig. 12 shows an example wherein a connect search is carried out again by designating a starting point molecule and an endpoint molecule in the once generated molecule network and the partial network obtained is marked.
Fig. 13 shows an example of the graphical user interface wherein information on pathological events represented hierarchically and the molecule network are operated interlinked with each other.
Fig. 14 shows an example of the graphical user interface wherein Gene Ontology which is the hierarchized information on bio-events and the molecule network are operated interlinked with each other.
Fig. 15 shows a concept of linking various biological information and related information to the molecule network.

### Detailed Explanation of Preferred Embodiments

In the following, embodiments of the present invention are explained specifically in relation to the drawings appropriately. However, the scope of the present invention is not limited by these explanations.

The meanings or the definitions of the terms in the present invention are as follows.

"Organism" is a concept including organelles, cells, tissues, organs and individuals, for example. Living entities such as bacteria, virus, and prion that infect an organism are also included in the concept of the organism.

"Bio-event" is a concept including all phenomena, responses, reactions, and symptoms appearing endogenously or exogenously in an organism. Specific examples include transcription, cell migration, cell adhesion, cell division, neural excitation, vasoconstriction, increase of blood pressure, decrease of blood glucose level, fever, convulsion, infection by parasites such as heterogeneous organisms and viruses. Responses to physical stimulations such as the light and heat from outside of an organism may be included in the concept of bio-event as well.

"Pathological event" is a concept that can be included in the "bio-event," and means a situation where a "bio-event" increases, decreases, or changes qualitatively, and which can be judged as a disease or a pathological state as a result. For example, as a consequence of an extraordinarily increased "bio-event" of blood pressure increase, high blood pressure or hypertension can be pointed out as the "pathological events," and when blood sugar exceeds a normal range, hyperglycemia or diabetes can be pointed out as the "pathological events." There are pathological events that are related to multiple kinds of bio-events, as well as the aforementioned examples that are related to a single bio-event. Moreover, information related to diseases such as disease names, symptoms and syndromes, clinical test items, disease classifications, and complications can be treated as the information on pathological events in a broader sense in the present invention.

"Molecule" includes nucleic acids, proteins, lipids, carbohydrates, organic compounds, inorganic compounds, metal ions, and others existing in an organism. Furthermore, molecules such as drug molecules, nutrients, environmental pollutants, poisons that exist outside of an organism or are taken from outside of an organism are included in the "molecule" of the present invention. "Biomolecule" includes molecules naturally existing in an organism and molecules from a foreign organism such as viruses or bacteria. "Physiologically active molecule" means a molecule that has one or more physiological activities, and is used as a concept including drug molecules.

"To correlate" means indicating or recording a direct or indirect relation between two or more data items selected from molecules, subnets, bio-events, pathological events, and genes. "Relation information" is the information recorded by "correlating" items. "Molecule Pair" means a set of two or more correlated molecules. A method of carrying out a connect search using a molecule and/or a molecule pair as a search point is described in detail, for example, in the specification of PCT/JP01/07830 or PCT/JP03/02847.

"Biological pathway" means a molecule network with a certain range called with a name. Hereinafter, it is sometimes referred to as "pathway" for simplicity. Examples of pathways include glycolytic pathway, citric acid cycle, MAP kinase cascade, and Caspase cascade. It should be understood that a molecule network with an arbitrary range may be treated as a pathway by giving a name for the sake of convenience.

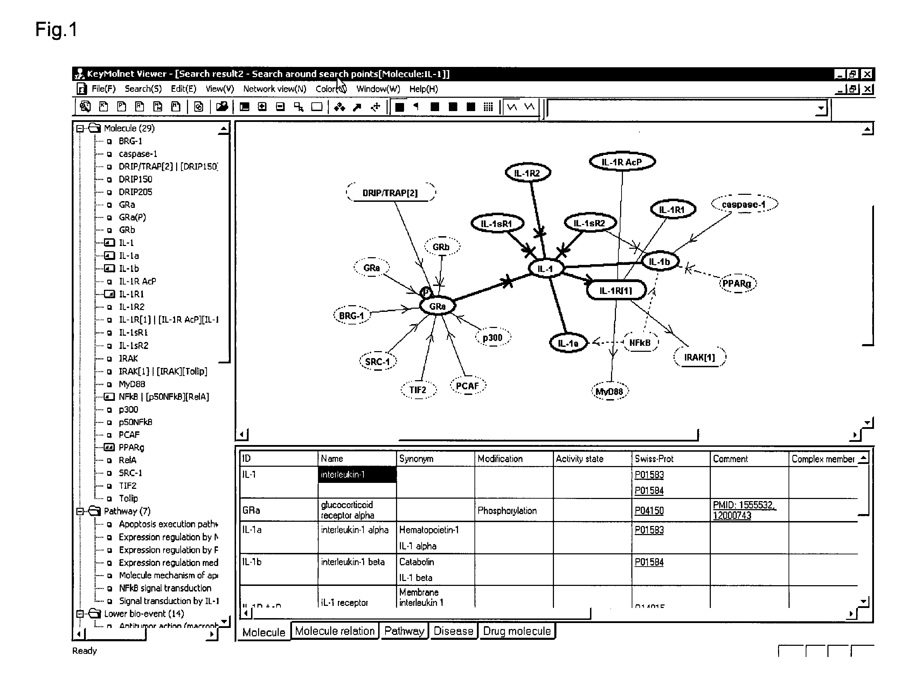

An example of the graphical user interface of the embodiment of the present invention is shown on Fig. 1. The graphical user interface of the present embodiment is characterized by that it includes a molecule network window to display a molecule network included in the molecule function network, and an information window to display one or more information items selected from a group comprising molecules, molecule pairs, and bio-events/pathological events (hereinafter, the expression "bio-event/pathological event" means both or either of the bio-event and the pathological event) included in the molecule function network.

Data of the molecule function network treated in the present embodiment can be prepared by the methods described in the specification of PCT/JP01/07830 or PCT/JP03/02847, for example. According to the methods described in these specifications of the PCT applications, a molecule function network with an arbitrary range including an arbitrary molecule, bio-event, or pathological event that a user has designated can be generated. As another embodiment, data of the molecule function network described in literatures or reviews that are expressed by an appropriate coding method can be used.

The molecule network window and the information window may be displayed as independent windows as shown in Fig. 1, or may be displayed so that one of the windows is included in the other window. Multiple molecule network windows and information windows may be displayed simultaneously. For example, detailed information may be displayed in one window, and summary information such as the title and the abbreviated symbols of the displayed items may be displayed in another information window. As a still further embodiment, one information window may be displayed for multiple molecule network windows.

In the molecule network window, a molecule network can be displayed as a graph wherein a molecule is an apex (an apex is sometimes called a "node") and molecules related with each other are linked with an edge. The apex representing a molecule may be displayed with a different symbol depending on the kind of the molecule, for example, an oval for a protein and a rectangle for a small molecule. The term apex does not merely mean a point, but can be interpreted in a broader sense to include a region having a certain area.

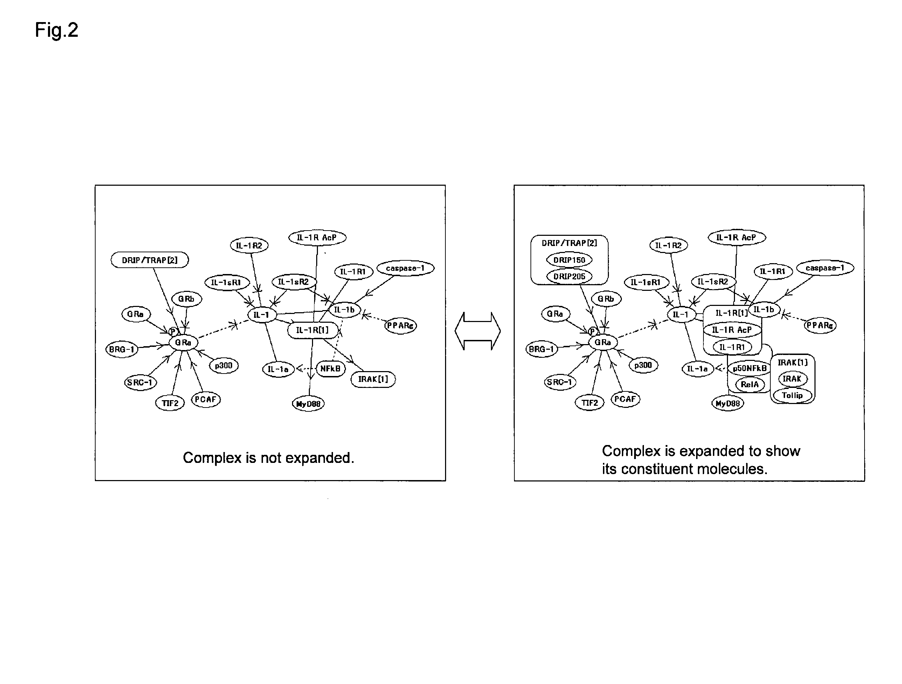

When multiple molecules form a complex and function as a unit, the complex may be displayed as an apex similarly for a single molecule. In this case, it is recommended to display the complex with a different symbol indicating the complex state. As shown in Fig.2, molecules constituting the complex may be displayed expanded within the symbol representing the complex. In a preferred embodiment of the graphical user interface of the present invention, a user can switch between an unexpanded display of the complex and an expanded display of the complex appropriately. The expanded display of a complex is not limited to that shown in Fig.2, and it should be understood that any expression may be used as long as the constituting elements of the complex can be distinctly shown.

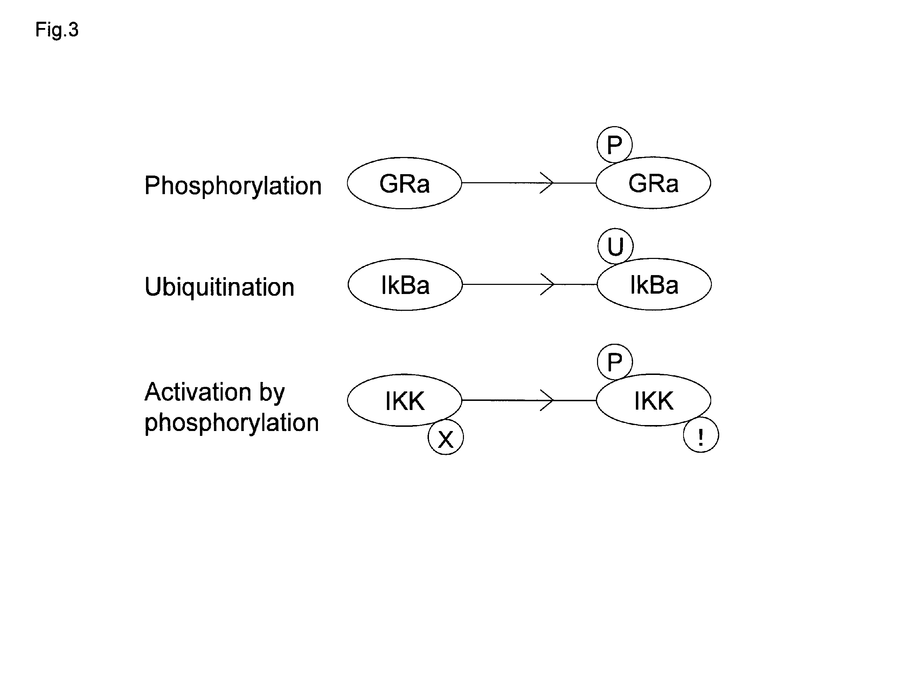

For the molecule whose relations with other molecules change by modifications such as the phosphorylation, different apexes may be used for respective modification states of the molecule for describing a molecule network. For the molecule whose relations with other molecules change between active and inactive states of the molecule, different apexes may be used for the different activation states of the molecule. In these cases, it is recommended to distinguish the modification states and/or the activation states, by appending a symbol representing the modification state and/or the activation state to the symbol representing the molecule as shown in Fig.3, or by displaying different kinds of symbols according to the modification state and/or the activation state. Fig.3 merely shows examples of such symbols, and it should be understood that any symbol may be used as long as a user can easily understand the difference of the modification states and/or the activation states.

As one of the preferred embodiments of the present invention, there is provided a method of displaying a molecule network wherein molecules and/or molecule pairs are displayed differently with symbols and/or colors based on the numeric information representing quantitative and/or qualitative states of the molecules and/or the molecule pairs. Examples of such numeric information include data of the amount of mRNAs measured by the experiment using DNA microarray (also called DNA chip), data of the amount of proteins measured by the proteomics experiment, and data of the metabolic product (mainly small molecules) measured by the metabolomics experiment. Not only the absolute values of the amounts of these substances, but also the ratios of the amounts of substances among two or more different states (for example, different cells or organs) may be used as the numeric data.

If the aforementioned numeric information is related to mRNAs, it is preferable to display the molecules corresponding to the proteins coded by the mRNAs with different colors. As a method other than color-coding, the size or the kind of the symbol representing the molecule in the molecule network may be changed depending on the numeric information. If the mRNAs or the proteins that have been measured are from a different species, it is possible to identify a protein in the molecule network corresponding to the mRNA or the protein that has been measured based on the ortholog information on relations of proteins between different species, and to color the identified protein based on the numeric information. Such methods for displaying a molecular network based on the numeric information are useful for a user to interpret the experimental data according to the molecule network.

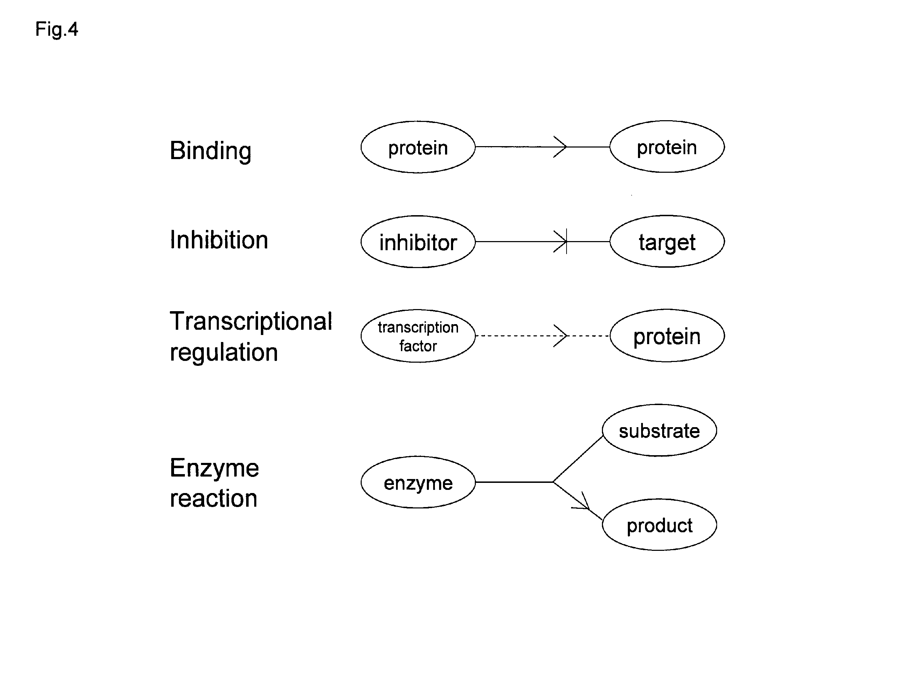

A method of drawing an edge between the related molecules (that is, molecule pairs) may be changed depending on the relation between the molecules. For example, as in Fig.4, it is possible to display the relation of binding between molecules with a solid arrow, the relation between an inhibiting molecule and its target molecule with a barred arrow, the relation between a transcription factor and the protein which is transcriptionally controlled with a dotted arrow, and the relation among the substrate, product and catalyzing enzyme involved in an enzyme reaction with a branched arrow. Preferably, the direction of the arrow may be decided according to the direction of the signal transduction. However, when the direction cannot be determined, a simple line without an arrow may be drawn. Besides the representation with the kinds of lines, line thickness and color-coding may be changed depending on the relation between the molecules.

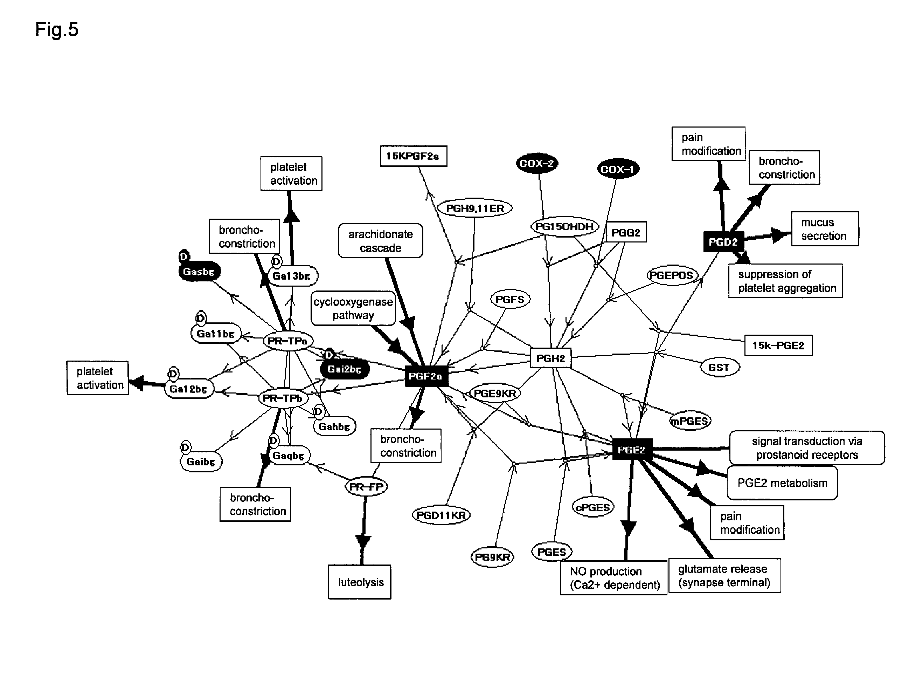

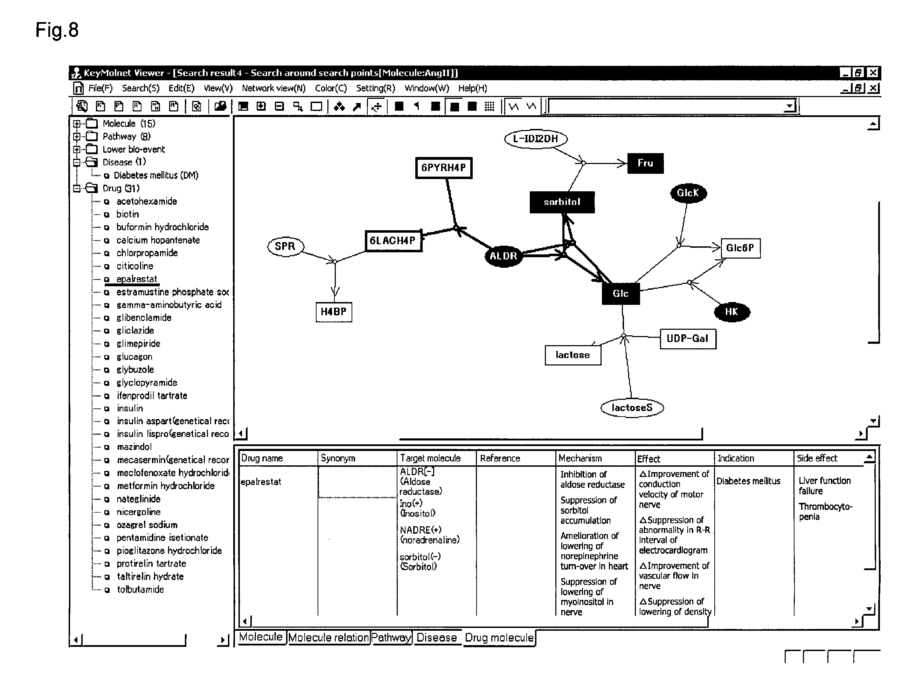

By using the different drawing methods for the edges between molecules according to the relations of the molecules as described above, molecule networks including different kinds of relations between molecules such as signal transduction, enzyme reaction, and expression control of protein by transcription factor can be displayed in a form easily understandable by a user, as shown in Fig. 1, Fig.5 and Fig.8. Moreover, the relations between proteins (displayed with ovals) and the conversion relations (metabolism) of small molecules (displayed with rectangles) by enzyme reactions can be displayed together in one molecule network, as shown in Fig.5 and Fig.8. It is one of the preferred embodiments of the present invention that a molecule network including different kinds of relations between molecules is displayed by using different methods for drawing edges between molecules depending on the relations of the molecules as described above.

It is one of the preferred embodiments of the present invention that different molecule networks are generated and displayed by controlling whether molecules are linked or not during a connect search according to the kinds of relations between molecules. For example, by using the relation of enzyme reactions as the relation between molecules for the connect search, a molecule network comprising only enzyme reactions such as the metabolic pathway and kinase cascade can be generated and displayed. By using only the relation between a transcription factor and the protein which is transcriptionally controlled as the relation between molecules, it is possible to generate and display a molecule network of the transcriptional control of proteins. Examples of the kinds of relations between molecules include enzyme reaction, activation, inactivation, inhibition, binding, expression induction of protein, and expression repression of protein. Classifications of the kinds of relations between molecules are not limited to these, and it should be understood that two or more kinds of relations between molecules may be used together.

Positions of molecules (apexes) constituting a molecule network may be determined interactively using an input device such as a mouse by a user. But more preferably, it is recommended to determine them by calculation so that the symbols representing the molecules are arranged at optimum positions with as little overlaps as possible. As an algorithm for the calculation, methods such as Force Directed Method and Layer Drawings (G. D. Battista et al., Graph Drawing-Algorithms for the Visualization of Graphs, Chapter 9 & 10, Prentice Hall, 1999) may be used, for example.

When a molecule network containing many molecules is generated by a connect search, there are cases that the molecule network graph calculated only by the optimum arrangement method as described above is too complicated for a user to understand. In such cases, the molecule network may be displayed as a "partitioned graph" by the following method. This method is characterized by that a graph of one molecule network is divided to multiple regions (hereinafter referred to as "partitions"), and nodes belonging to each partition is drawn with an optimum arrangement.

A node that is directly linked to a certain node in the molecule network graph is hereinafter referred to as an "adjacent node." Among nodes of a molecule network, nodes having at least a certain number of adjacent nodes are selected as the nodes to be centers of the partitions (hereinafter, referred to as "center nodes"). A center node and its adjacent nodes constitute a partition. After determining multiple partitions for the entire molecule network, the center nodes of respective partitions are located at optimum positions. For this purpose, the aforementioned calculation algorithm and simulated annealing method may be used.

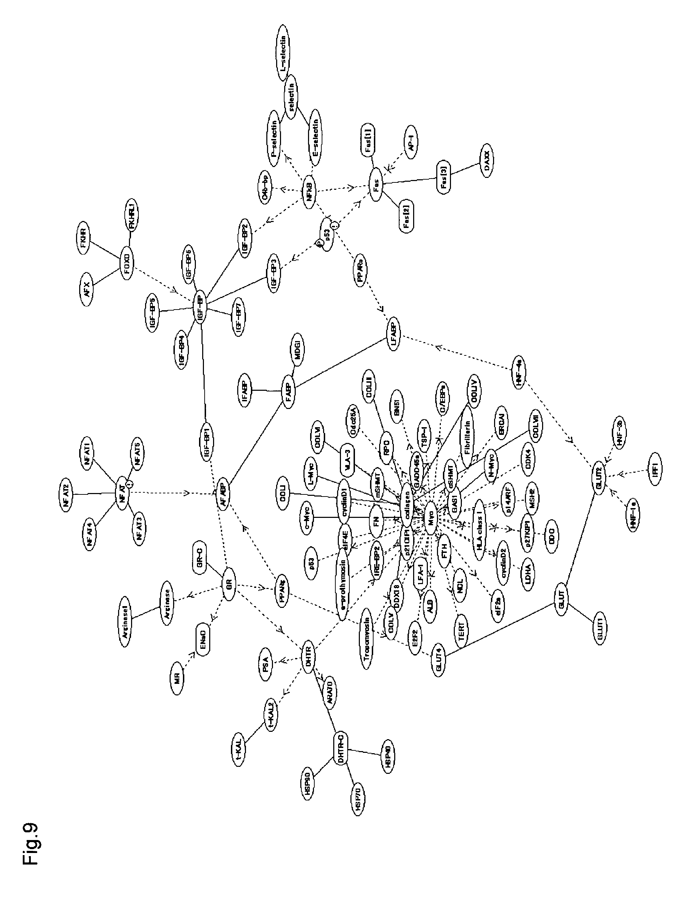

After determining a tentative arrangement of the center nodes as described above, adjacent nodes for each partition are optimally arranged. Finally, the arrangement of the center nodes of respective partitions and their adjacent nodes are readjusted while avoiding a collision between molecules belonging to different partitions. For this purpose, Force Transfer method can be used, for example. Fig.9 shows an example of a partitioned graph whose arrangement is determined by this way.

As one of the embodiments of the present invention, there is provided a method wherein the coordinates representing the arrangement of nodes of a molecule network graph displayed on the molecule network window are stored in a file, and the arrangement of the molecule network is reproduced upon the user's request. As another embodiment, there is also provided a method wherein a list of molecules (corresponding to the nodes of the graph) and molecule pairs (corresponding to the edges of the graph) constituting a molecule network graph is stored in a file, and the molecule network is reproduced without re-executing the connect search upon the user's request. Furthermore, by combining these two methods, it becomes possible to easily reproduce the molecule network that a user once generated and displayed while retaining its arrangement, when the user desires. A graphical user interface that executes these methods is one of the embodiments of the present invention.

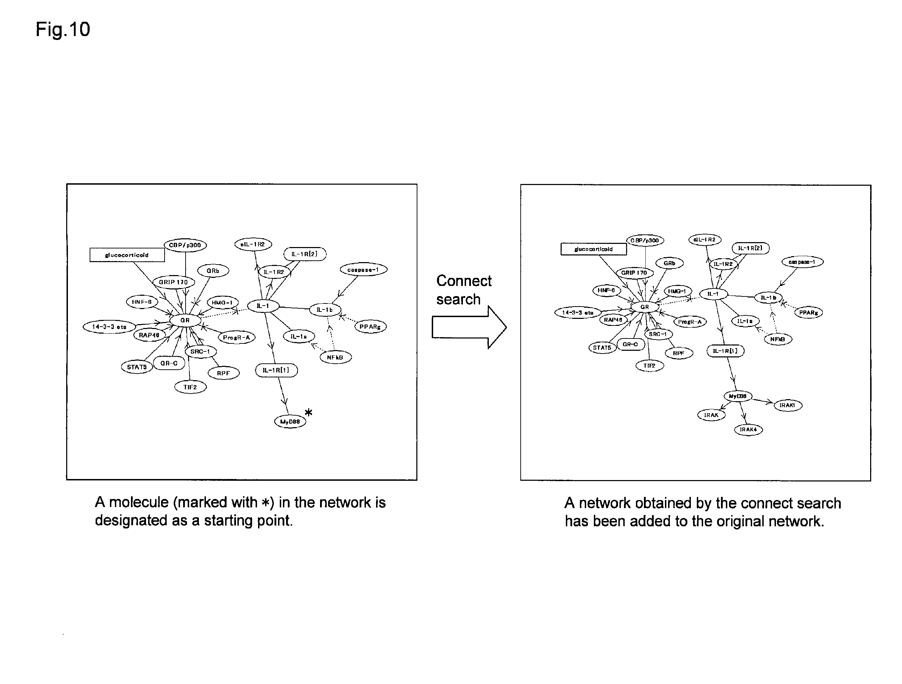

As one of the embodiments of the present invention, there is provided a graphical user interface characterized by that one or more molecules and/or molecule pairs in the molecule network window are selected, and a molecule network is generated and displayed by a connect search designating the selected molecule and/or the selected molecule pair as a search point. In this case, the molecule network generated by the connect search may be displayed in a new molecule network window, or displayed by adding to the original molecule network. By the addition to the original molecule network, it is possible for a user to expand the molecule network interactively and easily. In Fig. 10 shows an example of such operation of expanding a molecule function network.

A bio-event related to a molecule network may be added as an apex to the graph of the molecule function network. In this case, it is recommended to display the bio-event with a symbol different from those for molecules. For example, if a bio-event (hereinafter referred to as a downstream bio-event) is provoked by formation of a relation between certain molecules (that is, a molecule pair is formed), it is recommended to draw an arrow from the molecule pair (or either molecule of the molecule pair) to the downstream bio-event. When a relation between certain molecules is formed (that is, a molecule pair is formed) by an occurrence of a certain bio-event, it is recommended to draw an arrow from the bio-event (hereinafter, referred to as an upstream event) to the molecule pair (or either molecule of the molecule pair).

A pathway to which a molecule in a molecule network is related may be drawn as an apex to the graph of the molecule network. In this case, it is recommended to display the pathway with a symbol different from those for molecules. For example, if a pathway is activated by formation of a certain molecule pair, it is recommended to draw an arrow from the molecule pair (or either molecule of the molecule pair) to the pathway (hereinafter, referred to as a "downstream pathway"). When a certain molecule pair is formed by activation of a pathway, it is recommended to draw an arrow from the pathway (hereinafter referred to as an "upstream pathway") to the molecule pair (or either molecule of the molecule pair).

When a bio-event and/or a pathway is added to a molecule network graph, it is recommended to determine the graph arrangement by calculation so that the symbols representing the molecule and the bio-event and/or the pathway take an optimum arrangement with as little overlap as possible. Fig.5 shows an example of the display of a molecule network including bio-events and pathways. With such a representation, it becomes possible for a user to easily understand the relation between the molecule network and the bio-event and/or pathway in the molecule function network. Similar to the bio-event, pathological events may be added to the molecule network graph.

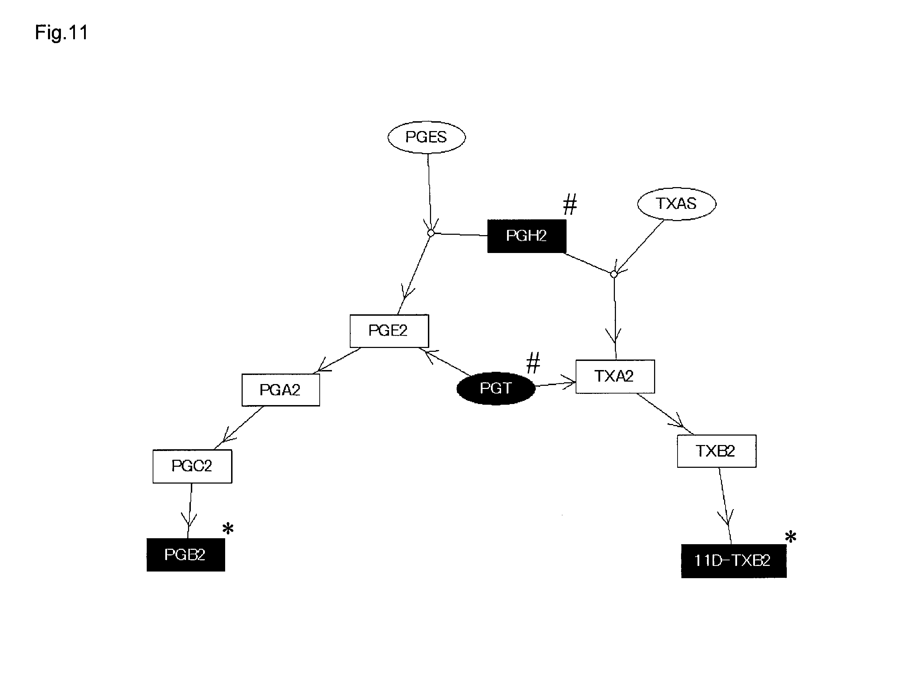

As another embodiment of drawing a graph of a molecule network, there is provided a method wherein a molecule network to molecules existing upstream or downstream in common to two or more molecules (hereinafter, referred to as "common upstream molecule" and "common downstream molecule," respectively) is generated by a connect search, and the common upstream molecule or the common downstream molecule is marked in the molecule network. Fig. 11 shows an example wherein a molecule network is generated by a connect search from two molecules to the common upstream molecules, and the common upstream molecules are marked. For marking the common upstream molecule or the common downstream molecule, a method of appending a symbol to the common upstream molecule or the common downstream molecule as in Fig. 11 and a method of displaying the molecules with different colors or symbols can be used.

A search for a common upstream molecule can be carried out by executing a connect search to the upstream direction (opposite direction to the arrow in Fig. 11) considering the direction of molecule pairs such as signal transduction, enzyme reaction, and transcriptional control from molecules designated as starting points, and by repeating the connect search recursively or successively until it reaches a common molecule. The common upstream molecule is not limited to one, and there are cases where two or more common upstream molecules are found as in Fig.11. A search for a common downstream molecule can be carried out by an identical method except that the direction of the molecule pair is considered oppositely.

By searching and displaying the common upstream molecule, a user can easily identify a molecule that affects the amounts or the states of multiple molecules in common. By searching and displaying the common downstream molecule, a user can easily identify a molecule whose amount or state is affected in common from multiple molecules. As described in the specification of PCT/JP03/02847, a connect search can be carried out using not only molecules but also a relation between arbitrary information items such as subnet, bio-event, pathological event, drug molecule, and gene. Thus, it should be understood that a search for the common upstream molecule and/or common downstream molecule can be executed regarding these information items other than molecules as the starting points.

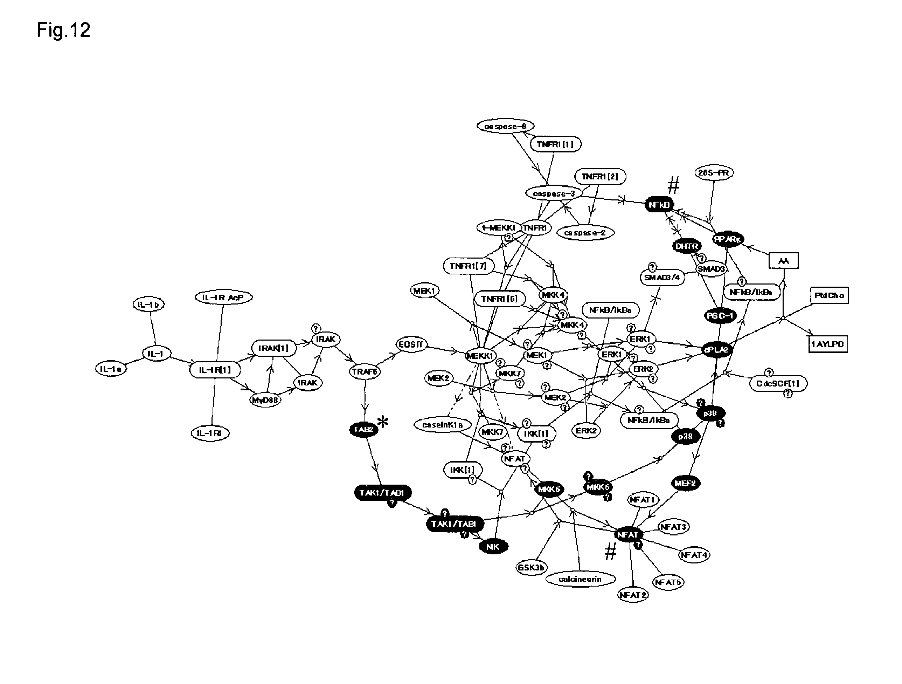

As another embodiment of drawing a graph of the molecule network, there is provided a method wherein a connect search is carried out again by designating one or more molecules in the molecule network generated before, and the molecule network generated therein (which is a partial network of the original molecule network) is marked in the original network. Fig. 12 shows an example wherein one molecule (indicated with the * mark in the figure) is designated the starting point, two molecules (indicated with the # mark in the figure) are designated as the end points, and a partial network connecting between these molecule is obtained by a connect search and marked in the original molecule network (molecules belonging to the partial network are shown in a reverse color). This method is useful for the purpose of predicting the range of affected molecule network when the expression of a certain protein is suppressed by the methods such as the knockout and the RNA interference (RNAi), and when the function of a certain protein are altered by the method of transgenic (also called as knockin).

The information window displays information on the molecules, molecule pairs, bio-events/pathological events included in the molecule function network being displayed. Although the form of displaying the information is not limited, it is preferable to display the information in a tabular form by category such as molecules, molecule pairs and bio-events (including pathological event). In the following examples, the information window is sometimes referred to as "molecule information window," "pathology information window" depending on the contents of the information displayed therein.

It is sometimes convenient that the information on bio-events is categorized by grouping and/or hierarchization depending on the relations among them. As an example of the hierarchization of information on bio-events, "Gene Ontology" by the Gene Ontology Consortium (http://www.geneonology.org) can be pointed out. As one of the preferred embodiments of the present invention, there is provided a graphical user interface wherein the hierarchized bio-events such as the Gene Ontology are displayed in the information window in a form such as a tree by which a user can easily comprehend the hierarchy, and the data items in the hierarchized information and the items in the molecule network window are operated interlinked with each other.

It is sometimes convenient as well, that the information on pathological events is categorized by grouping and/or hierarchization similar to the bio-events. Among the pathological events, as an example of the hierarchization of disease names, "International Classification of Diseases (ICD)" by the World Health Organization (WHO) can be pointed out. As examples of the hierarchization of pathological events including disease names and symptoms, MedDRA by the International Federation of Pharmaceutical Manufacturers Association (IFPMA) and SNOMED by the College of American Pathologists (CAP) can be pointed out. As one of the preferred embodiments of the present invention, there is provided a graphical user interface wherein the hierarchized information on the pathological events is displayed in the information window in a form such as a tree by which a user can easily comprehend the hierarchy, and the data items in the hierarchized information and the items in the molecule network window are operated interlinked with each other.

By quantifying the tightness of relation between a molecule network and information items such as pathways, bio-events, pathological events, and drug molecules by an appropriate method and by displaying the numeric values or markers indicating the tightness of relation to respective information items, it becomes possible for a user to understand the relations among information items easily and appropriately.

As an example of quantifying the tightness of relations, there is provided a method wherein the number of molecules correlated to a certain information item (any information item such as a pathway, a bio-event, a pathological event and a drug molecule may be used as long as it can be correlated to a molecule) among the molecules displayed in the molecule network window is counted, and the number (hereinafter referred to as "correlated molecule number") is used as an index of the tightness of the relation.

If information on each correlation includes a weight representing the tightness of the relation, the weights may be summed up when the number of molecules is counted, or as another method, it may be accepted to judge whether to count the molecules or not based on the weight. The correlated molecule number itself may be used as an index, or as another method, a ratio obtained by dividing the correlated molecule number by any one of the numbers listed below may be used as an index; the number of all molecules displayed in the molecule network, or the number of molecules correlated to the information item among the molecules in the database of the present invention.

By displaying the index of the tightness of relation thus calculated for each information item in the information window, it becomes possible for a user to understand easily how tightly a certain information item and the displayed molecule network are related. Besides the method of displaying the index, a method of adding an appropriate marker according to the index or a method of coloring or highlighting the information items may be used.

One of the characteristics of the preferred embodiments of the present invention is that related items in the molecule-network window and in the information window can be operated interlinked with each other. Although specific examples are given below, it should be understood that the scope of the present invention is not limited to the following examples.

### Examples

In the following examples, it is assumed that "biomolecule information database," "biomolecule-linkage database," "drug molecule information database," and "pathology-linkage database" are provided. In the biomolecule information database, information related to biomolecules is stored. Examples of the information to be stored include molecule ID, synonym, structure code, function code, species, originating organ, and existing organ. In the biomolecule-linkage database, information on pairs of biomolecules related to each other is stored. Examples of the information to be stored include the formal names and molecule IDs of two molecules constituting a pair, a condition for the formation of the relation, and a bio-event caused by the relation.

In the "drug molecule information database," information related to drug molecules is stored. Examples of the information to be stored include names of drug molecules, molecule IDs, indication, and target biomolecules. In the "pathology-linkage database," information related to diseases is stored. Examples of the information to be stored include names of diseases, key molecules that change qualitatively or quantitatively in respective diseases, pathological events, symptoms and syndromes, complications, disease classification, and clinical test items. It is desirable not only to store the items of each category, but also to store information on the relation between items belonging to different categories.

As methods for constructing the above-mentioned databases, and methods for generating a molecule function network using those databases, the methods described in the specification of PCT/JP01/07830 or PCT/JP03/02847 may be used.

### Example 1

In Fig. 1, an example of displaying molecule items in the molecule network window and in the information window interlinked with each other. First, the system generates a molecule function network with an appropriate range according to the designation by a user. In the molecule network window, a molecule network is displayed according to the above-mentioned methods. When the user clicks an arbitrary apex (corresponding to a molecule) or an edge (corresponding to a relation between molecules) in the molecule network window, the system searches the molecule information database using the corresponding molecule(s) as a query, and the obtained molecule information is displayed in the information window. In Fig.1, the molecule information is displayed in the information window at bottom-right of the screen by grouping the molecule information into different columns for respective categories so that the user can view the information easily. With this display method, the user can easily view the information on an arbitrary molecule in the molecule network.

When an edge is clicked in the above example, it is preferable that the molecule information database is searched for the molecules of the both ends of the edge, and the search result is merged and displayed in the information window. It is also acceptable that the neighborhood of the clicked apex or edge is searched based on the topology of the molecule network to obtain a list of molecules topologically connected within a certain range, the molecule information database is searched for the molecules in the list, and the search result is merged and displayed in the information window. Fig.1 shows an example wherein the information on the molecules that are within one path from the clicked molecule (IL-1) is displayed in the information window at bottom-right of the screen. With this display method, the user can easily view the information on the molecules in the neighborhood of an arbitrary molecule in the molecule network.

By highlighting the molecule or edge that the user has clicked with a different color or with a different line width in the molecule network window, the user can easily understand the relation between the clicked molecule or edge and the contents displayed in the information window. When the neighboring molecules of the clicked molecule or edge are searched based on the topology, it is recommended to highlight not only the clicked molecule but also the neighboring molecules as shown in the molecule network at upper-right of Fig. 1.

Searching of the molecule information database of this example may be carried out using all entries of the database. If a subset of the molecule information database that contains the molecules included in the generated network has been extracted at the time of generation of the molecule function network, the searching can be carried out more rapidly using the subset of the database.

Furthermore, using the above subset of the molecule information database, information on all molecules contained in the molecule network may be displayed in the molecule information window at the time of displaying the molecule network in the molecule network window. In this case, it is preferable that when the user clicks an apex or an edge in the molecule network window, the molecule information is searched similarly as above and the corresponding molecule information is highlighted in the information window. With this highlighting, the user can easily view the information on arbitrary molecules in the molecule network. On the contrary, it is preferable that when the user clicks an item in the molecule information window, the corresponding molecule is highlighted in the molecule network window.

### Example 2

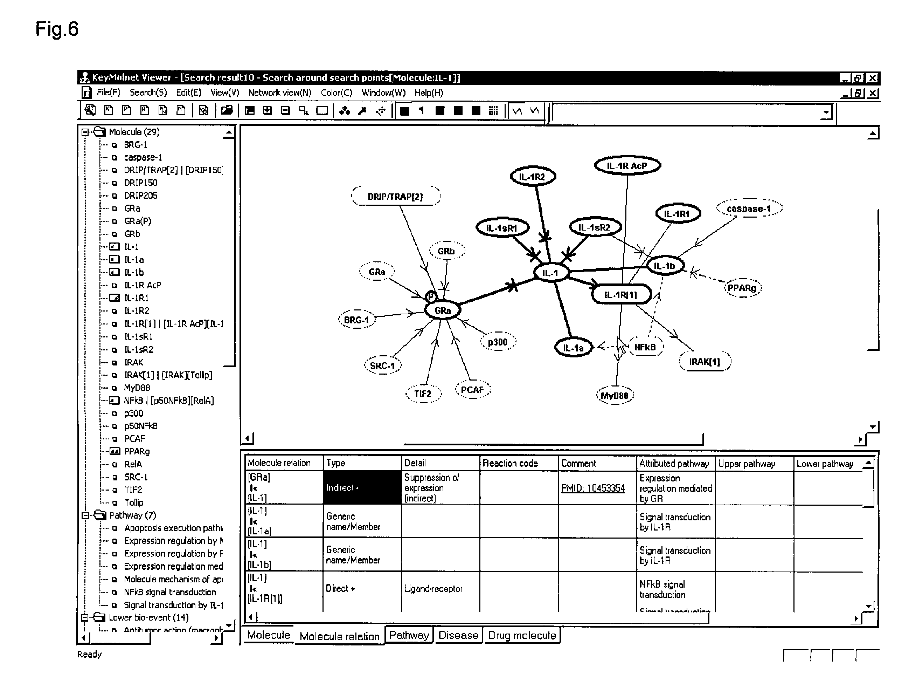

Fig.6 shows an example of displaying molecule-pair items in the molecule network window and in the information window interlinked with each other. First, the system generates a molecule network with an appropriate range according to the designation by a user. In the molecule network window, a molecule network is displayed according to the above-mentioned methods. When the user clicks an arbitrary edge (corresponding to a relation between molecules) in the molecule network window, the system searches a molecule pair consisting of molecules of the both ends of the edge from the molecule-linkage database, and displays information on the obtained molecule pair in the information window.

When the user clicks an arbitrary molecule in the molecule network window, it is preferable that the system searches a molecule pair containing the clicked molecule at either end from the molecule-linkage database, and displays information on the obtained molecule pair in the information window. If molecules in the neighborhood of the clicked molecule or edge is searched based on the topology by the method of Example 1, it is preferable that the system searches molecule pairs containing the neighboring molecules from the molecule-linkage database, and displays information on the obtained molecule pairs in the information window.

The method of Example 1 of highlighting the molecule or edge clicked in the molecule network window can be used also in this example. With the method of highlighting, the user can easily understand the relation between the clicked molecule or edge and the contents displayed in the molecule-pair information window. In the example of Fig.6, information on molecule pairs (described as "molecule relation information" in the figure) between IL-1, which the user has clicked, and the molecules that are within one path from IL-1 is displayed in the information window at right-bottom.

Searching of the molecule-linkage database of this example may be carried out using all entries of the database. If a subset of the molecule-linkage database that contains only the molecules included in the molecule network has been extracted at the time of the generation of the molecule function network, the searching can be carried out more rapidly using the subset of the database.

Furthermore, using the above subset of the molecule-linkage database, information on all molecule pairs included in the molecule network may be displayed in the molecule-pair information window at the time of displaying the molecule network in the molecule network window. In this case, it is preferable that when the user clicks an apex or an edge in the molecule network window, molecule pairs are searched similarly as above and the information on the obtained molecule pairs is highlighted in the information window. With this highlighting, the user can easily view the information on arbitrary molecule pairs in the molecule network. On the contrary, it is preferable that when the user clicks an item in the molecule-pair information window, the corresponding edge is highlighted in the molecule network window.

### Example 3

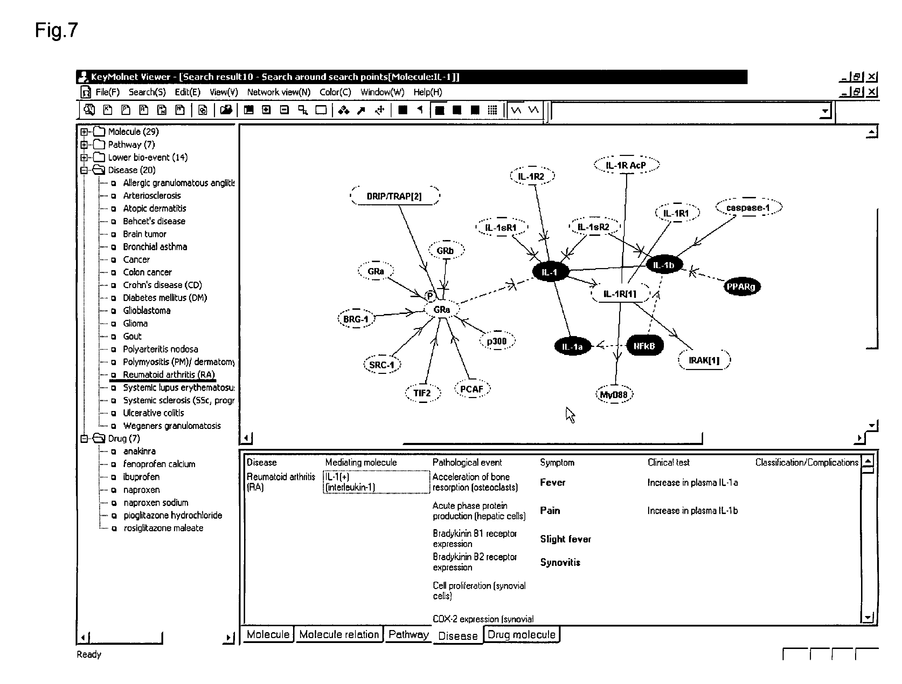

Fig.7 shows an example of displaying items of the pathological events in the molecule network window and in the information window interlinked with each other. In the display of the molecule network at upper-right, the key molecules that change quantitatively or qualitatively in diseases are displayed with black-and-white reversed (molecules such as IL-1, NFkB, and PPARg) based on the information on key molecules in the pathology-linkage database.

When the user clicks IL-1, which is one of the key molecules, on the molecule network display, the system highlights a disease ("rheumatoid arthritis" which is underlined) related to the key molecule (that is, IL-1) in the summary information window on the left based on the information in the pathology-linkage database. At the same time, the system extracts information on the disease from the pathology-linkage database and displays items in the detailed information window at bottom-right. Here, all information related to the disease may be displayed, but it is convenient that only those items related to the clicked key molecule are displayed so that the relation between the key molecule and the information items is easily understandable. Fig.7 is an example of displaying only the items related to IL-1. It shows, for example, that the key molecule IL-1 (shown as "mediating molecule" in the figure) has relations with pathological events such as "COX-2 expression (synovial cell)" and "synovitis."

### Example 4

Fig.8 shows an example of displaying items of the drug molecule information in the molecule network window and in the information window interlinked with each other. In the display of the molecule network at upper-right, the biomolecules that are the targets of the actions of drug molecules are displayed with black-and-white reversed (molecules such as ALDR and HK), based on the information on target biomolecules in the drug molecule information database.

When the user clicks ALDR, which is one of the target biomolecules, on the molecule network, the system highlights a drug ("epalrestat" which is underlined) that targets the target biomolecule (that is, ALDR) in the summary information window on the left based on the information in the drug molecule information database. At the same time, the system extracts information related to the drug molecule from the drug molecule information database, and displays items in the detailed information window at bottom-right.

### Example 5

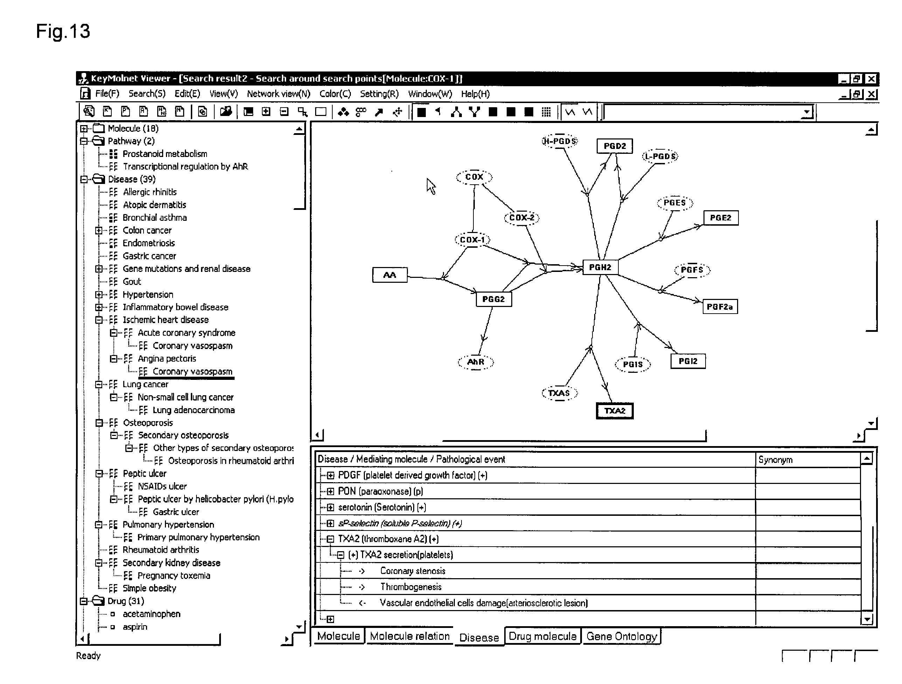

Fig. 13 shows an example of a graphical user interface that operates information on the pathological events represented hierarchically and a molecule network interlinked with each other. The system displays information on disease names among the pathological events hierarchically in the information window on the left. When the user clicks an item in this window, for example, "Ischemic heart disease / Angina pectoris / Coronary vasospasm", the system searches key molecules corresponding to the disease name based on the information in the pathology-linkage database, and highlights TXA2 molecule, among the key molecules, which is included in the molecule network window at upper-right.

At the same time, the system displays a list of key molecules corresponding to the disease name in the information window at lower-right, and displays the type of change of TXA2 molecule observed in the disease (+ indicates increase or activation) and the information on pathological events related to the change hierarchically. The causal relations between the change of the key molecule and the pathological events are displayed with a rightward arrow or a leftward arrow. By displaying the information on the pathological events represented hierarchically and the molecule network interlinked with each other like this, the user can easily view and understand the relation between the information on the pathological event and the molecules in the molecule network.

### Example 6

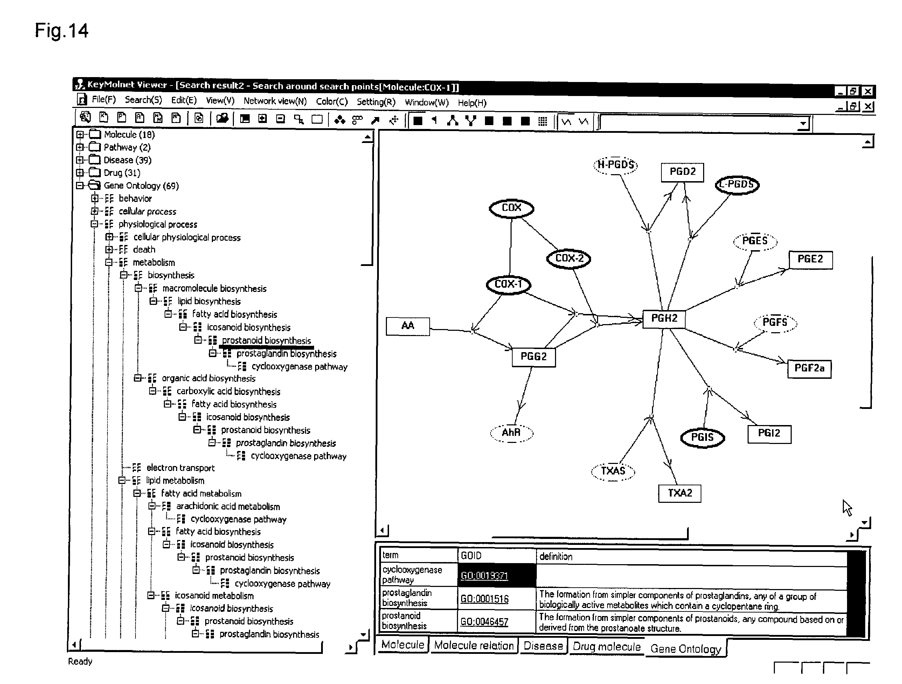

Fig. 14 shows an example of a graphical user interface that operates information on Gene Ontology, which is a kind of hierarchized representation of bio-events, and the molecule network interlinked with each other. The system displays the Gene Ontology information hierarchically in the information window on the left. When the user clicks an item in this window, for example, "prostanoid biosynthesis", the system searches molecules corresponding to the item based on the information in the biomolecule-linkage database, and highlights the molecules (COX, COX-1, COX-2, L-PGDS, PGIS), among the corresponding molecules, that are included in the molecule network window at upper-right.

At the same time, the system displays detailed information on the bio-events (items of the Gene Ontology) in the information window at bottom-right. Here, not only the clicked item (prostanoid biosynthesis), but also the items registered at lower hierarchies in the Gene Ontology are also displayed. By operating the information on bio-events represented hierarchically and the molecule network interlinked with each other, the user can easily view and understand the relation between the information on the bio-events and the molecules in the molecule network.

In this example, the tightness of relation between each item of the bio-event and the displayed molecule network is shown with small black dots to the left of each item of the bio-event. For example, to the left of the item "prostanid biosynthesis", two and three black dots are displayed in two columns, respectively. The number of the black dots in the left column represents the ratio of molecules related to the bio-event among all molecules in the molecule network, and the number of the black dots in the right column represents the ratio of molecules displayed in the molecule network among all molecules related to the bio-event. The number of black dots displayed is appropriately adjusted according to the value of the ratio.

### Industrial Applicability

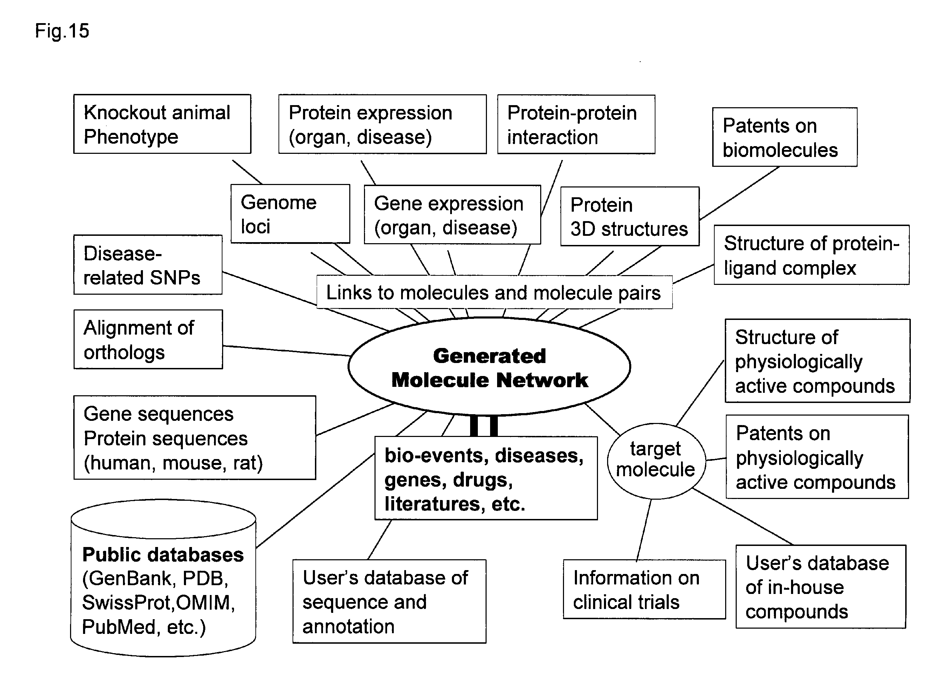

With the methods and graphical user interfaces of the present invention, it becomes possible to view various biological information such as biomolecules, bio-events, pathological events, drug molecules, and genes easily in connection with the molecule function network. Fig. 15 shows a concept of connections between the molecule network and various biological information and related information that can be treated by the methods and graphical user interfaces of the present invention.

## Claims

1. A graphical user interface wherein a molecule function network with an arbitrary range is generated according to the designation by a user, and the molecule function network is displayed graphically.

2. A graphical user interface comprising a molecule network window that displays a molecule network included in the molecule function network and an information window that displays one or more information items selected from a group comprising molecules, molecule pairs, and bio-events included in the molecule function network, and **characterized by** that items related with each other in the molecule-network window and in the information window are operated interlinked with each other.

3. A graphical user interface **characterized by** that a molecule pair in the molecule-network window and information on the bio-event which occurs due to a quantitative and/or a qualitative fluctuation of the molecule pair or which causes a quantitative and/or a qualitative fluctuation of the molecule pair are displayed interlinked with each other, and that the displayed items are operated interlinked with each other.

4. A graphical user interface **characterized by** that a molecule in the molecule-network window and information on the bio-event which occurs due to a quantitative and/or a qualitative fluctuation of the molecule or which causes a quantitative and/or a qualitative fluctuation of the molecule are displayed interlinked with each other, and that the displayed items are operated interlinked with each other.

5. A graphical user interface **characterized by** that a molecule in the molecule-network window and information on the drug and/or physiologically active molecule which acts on the molecule are displayed interlinked with each other, and the displayed items are operated interlinked with each other.

6. A graphical user interface with a window to display a list of molecules in the molecule-network window, which is **characterized by** that the molecules in the molecule-network window and items in the list window are operated interlinked with each other.

7. A graphical user interface with a window to display a list of information items related to molecules and/or molecule pairs in the molecule-network window, which is **characterized by** that the molecules and/or molecule pairs in the molecule-network and the items in the list window are operated interlinked with each other.

8. A graphical user interface with a window to display a list of biological pathways to which molecules and/or molecule pairs in the molecule-network window belong, which is **characterized by** that the molecules and/or molecule pairs in the molecule-network window and the items in the list window are operated interlinked with each other.

9. A graphical user interface with a window to display a list of information on bio-events related to molecules and/or molecule pairs in the molecule-network window, which is **characterized by** that the molecules and/or molecule pairs in the molecule-network window and the items in the list window are operated interlinked with each other.

10. A graphical user interface with a window to display a list of information on pathological events, which is **characterized by** that items related with each other in the list window are operated interlinked with each other.

11. The graphical user interface of claim 10 **characterized by** displaying the pathological events by category.

12. The graphical user interface of claim 11 comprising a list of information on quantitative and/or qualitative fluctuations of biomolecules related to the pathological events.

13. A graphical user interface **characterized by** that information on the molecule function network is searched by a keyword, and the item hit by the search is highlighted in the molecule network window and/or in the related list window.

14. A graphical user interface **characterized by** that one or more molecules and/or molecule pairs are selected in the molecule network window, and a molecule function network is generated and displayed by a connect search by designating the selected molecules and/or molecule pairs as search points.

15. A method of displaying a molecule function network, which is **characterized by** displaying molecules and/or molecule pairs differently with symbols and/or colors based on the information on the modification state and/or the activation state.

16. A method of displaying a molecule function network, which is **characterized by** displaying molecules and/or molecule pairs differently with symbols and/or colors based on the information on the bio-events.

17. A method of displaying a molecule function network, which is **characterized by** displaying molecules and/or molecule pairs differently with symbols and/or colors based on the information on the biomolecules that are the target of actions of physiologically active molecules.

18. A method of displaying a molecule function network, which is **characterized by** displaying molecules and/or molecule pairs differently with symbols and/or colors based on the numeric information representing quantitative and/or qualitative states of the molecules and/or the molecule pairs.

19. A method of displaying a molecule function network, which is **characterized by** displaying edges connecting molecule pairs differently by different drawing methods based on the relation information of the molecule pairs.

20. A method displaying a molecule function network, which is **characterized by** that representation of a complex with two or more biomolecules can be switched between a single symbol for the complex and multiple symbols for respective molecules constituting the complex.

21. A method of displaying a molecule function network, which is **characterized by** that a bio-event related to a molecule and/or a molecule pair in the molecule network is displayed as an apex, and the molecule and/or molecule pair and the apex are connected with an edge.

22. A method of displaying a molecule function network, which is **characterized by** that a biologically active molecule which targets a molecule and/or a molecule pair in the molecule network is displayed as an apex, and the molecule and/or molecule pair and the apex are connected with an edge.

23. A method of displaying a molecule function network, which is **characterized by** that a biological pathway and/or another molecule network related to a molecule and/or a molecule pair in the molecule network is displayed as an apex, and the molecule and/or molecule pair and the apex are connected with an edge.

24. A display grogram of the molecule function network that executes the graphical user interface described in any one of the claims 1 to 14.

25. A display grogram of the molecule function network that executes the display method described in any one of the claims 15 to 23.

26. A computer-readable medium recording the program of claim 24 or 25.

27. A device for displaying a molecule function network whereupon the program of claim 24 or 25 can be executed.
